# EUROPÄISCHE PATENTSCHRIFT

(11) **EP 0 815 082 B1**
(45) Veröffentlichungstag und Bekanntmachung des Hinweises auf die Patenterteilung: **16.01.2002**
(21) Anmeldenummer: 96908047.2
(22) Anmeldetag: 15.03.1996
(51) Int. Cl.: C07D 211/94, C07B 63/04

(54) **4-ACYLAMINOPIPERIDIN-N-OXYLE**
4-ACYLAMINO PIPERIDIN-N-OXYLS
4-ACYLAMINOPIPERIDIN-N-OXYLES

(30) Priorität: 21.03.1995 DE 19510184
(43) Veröffentlichungstag der Anmeldung: 07.01.1998
(73) Patentinhaber: BASF AKTIENGESELLSCHAFT, 67056 Ludwigshafen (DE)
(72) Erfinder: KOCH, Andreas, D-67240 Bobenheim-Roxheim (DE); AUMÜLLER, Alexander, D-67435 Neustadt (DE); MITULLA, Konrad, D-67071 Ludwigshafen (DE); TREMMEL, Gregor, D-67269 Grünstadt (DE); HERBST, Holger, D-67227 Frankenthal (DE)
(86) Internationale Anmeldenummer: EP9601122
(87) Internationale Veröffentlichungsnummer: WO9629311

(56) Entgegenhaltungen:
- EP-A- 0 094 048
- EP-A- 0 316 582
- EP-A- 0 581 737
- EP-A- 0 697 386
- CHEMICAL ABSTRACTS, vol. 99, no. 26, 26.Dezember 1983 Columbus, Ohio, US; abstract no. 213025t, M. D. GOLDFEIN ET AL.: "Stabilization of styrene or methyl methacrylate" Seite 8; Spalte 2; XP002006988 in der Anmeldung erwähnt & SU,A,1 027 150 7.Juli 1983
- MACROMOLECULES, Bd. 23, Nr. 3, 5.Februar 1990, EASTON US, Seiten 946-947, XP002006983 E.J.VLIETSTRA ET AL.: "Synthesis and Magnetic Properties of a Rigid High Spin Density Polymer with Piperidine-N-oxyl Pending Groups"
- INVESTIGATIVE RADIOLOGY, Bd. 21, Nr. 2, Februar 1986, Seiten 125-131, XP000574693 RICHARD L. EHMAN ET AL.: "Diradical Nitroxyl Spin Label contrast Agents for magnetic Resonance Imaging. A Comparison of Relaxation Effectiveness "
- JOURNAL OF THE AMERICAN CHEMICAL SOCIETY, Bd. 99, Nr. 5, 2.März 1977, DC US, Seiten 1637-1642, XP002006984 PAUL REY ET AL.: "Clustering of Nitroxide Spin Labels in Lipid Bilayer Membranes"
- JOURNAL OF ORGANIC CHEMISTRY, Bd. 56, Nr. 21, 11.Oktober 1991, Seiten 6110-6114, XP000229776 ZHENKUN MA ET AL: "Organic Oxoammonium Salts. 3.1 A New Convenient Method for the Oxidation of Alcohols to Aldehydes and Ketones"
- BULLETIN OF THE ACADEMY OF SCIENCES OF THE USSR DIVISION OF CHEMICAL SCIENCE., Bd. 34, Nr. 8, 20.Februar 1986, NEW YORK US, Seiten 1700-1704, XP002006985 G. N. BONDAREV ET AL.: "Synthesis of 2,2,6,6-Tetramethyl-4-Diaminoalkylpiperi- din-1-oxyls"
- BULLETIN OF THE ACADEMY OF SCIENCES OF THE USSR DIVISION OF CHEMICAL SCIENCE., Bd. 35, Nr. 2, 20.August 1986, NEW YORK US, Seiten 363-367, XP002006986 G. N. BONDAREV ET AL. : "Synthesis of 2,2,6,6-Tetramethyl-4-Amino-N-(Alkylmaleim ido)-Piperidyl-1-Oxides"
- BULLETIN OF THE ACADEMY OF SCIENCES OF THE USSR DIVISION OF CHEMICAL SCIENCE., Bd. 37, Nr. 7, 20.Januar 1989, NEW YORK US, Seiten 1455-1457, XP002006987 G. N. BONDAREV ET AL.: "Synthesis of 2,2,6,6-Tetramethyl-4-Amino-N-(Alkylaminod ichlortriazine)-1-Oxylpiperidines"
- DATABASE CROSSFIRE BEILSTEIN INFORMATIONSSYTEME GMBH, FRANKFURT DE XP002006989 & CAN.J.CHEM., Bd. 62, 1984, Seiten 1446-1457,
- DATABASE CROSSFIRE BEILSTEIN INFORMATIONSSYSTEME GMBH, FRANKFURT DE XP002006990 & CHEM.PHYS.LIPIDS, Bd. 7, 1971, Seiten 207-213,
- DATABASE CROSSFIRE BEILSTEIN INFORMATIONSSYTEME GMBH, FRANKFURT DE XP002006991 & BULL.ACAD.SCI.USSR DIV.CHEM.SCI., 1968, Seite 2106
- DATABASE CROSSFIRE BEILSTEIN INFORMATIONSSYSTEME GMBH, FRANKFURT DE XP002006992 & BULL.ACAD.SCI.USSR DIV.CHEM.SCI., 1969, Seite 1100

## Beschreibung

Die vorliegende Erfindung betrifft neue 4-Acylaminopiperidin-N-oxyle
der allgemeinen Formal Ia

A¹B¹ (Ia)

in der A¹ für einen organischen Rest ausgewählt aus der Gruppe bestehend aus
C₁-C₂₂-Alkyl,
C₃-C₂₂-Alkenyl,
C₃-C₁₂-Cycloalkyl,
durch Cyan, Hydroxy oder Carboalkoxy substituiertes C₂-C₂₂-Alkyl,
durch Ethersauerstoff oder Stickstoff unterbrochenes oder durch Hydroxy substituiertes C₄-C₂₂-Alkyl,
substituierte C₇-C₂₂-Phenyl- und C₁₃-C₂₂-Diphenylalkylreste,
Arylreste,
Heterocyclen tragendes C₁-C₂₂-Alkyl und
phosphorhaltige Gruppen
und B¹ für einen Rest der allgemeinen Formel IIa steht 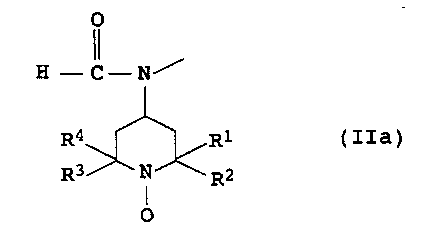 in welcher die Substituenten folgende Bedeutung haben:
   - R¹-R⁴: C₁-C₄-Alkyl, wobei R¹ und R² sowie R³ und R⁴ auch zu einem 5- oder 6-gliedrigen Ring verbunden sein können,
ausgenommen die Verbindung 4-[N-Formyl-N(3-hydroxypropyl)amino]-2,2,6,6-tetramethylpiperidin-1-oxyl, und
der allgemeinen Formel Ib

   A²(B²)ₙ (Ib)
in der A² für einen n-wertigen organischen Rest ausgewählt aus der Gruppe bestehend aus
C₂-C₂₂-Alkylen und C₅-C₂₂-Cycloalkylen,
C₈-C₁₄-Phenylalkylen und Phenylen,
durch Ethersauerstoff, Stickstoff oder Heterocyclen unterbrochenes Alkylen und
kohlenstoff-, sauerstoff- und/oder stickstoffhaltige Brücken mit Phosphor als Heteroatom steht,
n den Wert 2 bis 4 hat und B² mindestens einen der Reste IIa und die übrigen Reste B² gleiche oder verschiedene Reste der Formel IIb 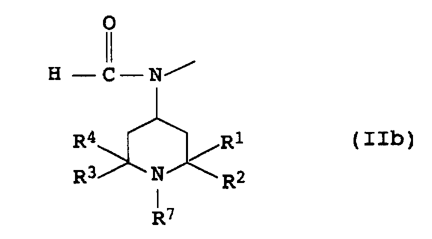
bedeuten, wobei R⁷ Wasserstoff, die Hydroxygruppe oder Formyl, O-Alkyl, O-Aryl, O-Hydrocarbyl, O-Carbamoyl, Cyanmethyl oder substituiertes Alkenyl bedeutet.

Außerdem betrifft die Erfindung ein Verfahren zur Herstellung der Verbindungen Ia, die Verwendung der Verbindungen zur Stabilisierung organischer Materialien gegen die schädigende Wirkung von Radikalen, besonders von Styrol während der Destillation, die gemeinsame Verwendung der Verbindungen Ia bzw. Ib mit aromatischen Nitro- oder Nitrosoverbindungen oder substituierten Phenolen sowie Kunststoffe, Lacke, Öle, Fette und radikalisch polymerisierende Monomere, welche die Verbindungen Ia enthalten.

Die Stabilisierung organischer Materialien gegen Schädigung durch Radikale, wie sie unter dem Einfluß von Licht oder Wärme entstehen, ist allgemein bekannt. Als Stabilisatoren wurden bisher für diesen Zweck Verbindungen verschiedener Stoffklassen vorgeschlagen, darunter die N-Oxyle verschiedener Derivate von 2,2,6,6-tetramethylierten Piperidinen.

Eine Verbindung dieser Art, die sich vom 4-Amino-2,2,6,6-tetramethylpiperidin-N-oxyl ableitet, ist das Bisamid der Adipinsäure 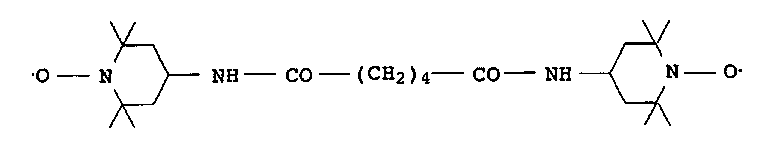 welches in der SU-A 1 139 722 beschrieben wird. Ähnliche bekannte Verbindungen enthalten statt der Carbonamidgruppierung die Ester- oder Carbamatgruppierung (EP-A 0 581 737 bzw. SU-A 1 027 150).

Weiterhin ist es aus der EP-A 0 581 737 bekannt, daß die stabilisierende Wirkung von N-Oxylen erhöht wird, wenn man sie zusammen mit aromatischen Nitroverbindungen anwendet.

Außerdem sind aus der EP-A 0 316 582 nicht-radikalische Pipendinderivate mit der Struktureinheit 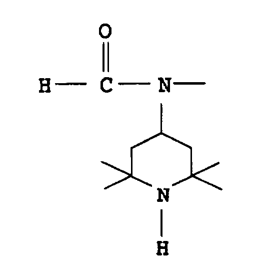 bekannt, die sich als Stabilisatoren für organisches Material eignen.

Da die bisher bekannten N-Oxyle in ihrer Wirkung zu wünschen übrig lassen, lagen der Erfindung neue N-Oxyle mit verbesserten anwendungstechnischen Eigenschaften als Aufgabe zugrunde.

Demgemäß wurden die eingangs definierten 4-Acylaminopiperidin-N-oxyle gefunden.

Weiterhin wurde ein Verfahren zur Herstellung der Verbindungen Ia bzw. Ib, die Verwendung dieser Verbindungen zur Stabilisierung organischer Materialien, ihre gemeinsame Verwendung mit anderen Stabilisatoren und Kunststoffe, Lacke, Öle, Fette und radikalisch polymerisierende Monomere, welche die Verbindungen Ia bzw. Ib enthalten, gefunden.

Das für die stabilisierenden Eigenschaften wesentliche Strukturelement der Verbindungen Ia ist der Molekülteil B¹ der allgemeinen Formel IIa.

In dieser Formel können R¹ bis R⁴ Alkylreste wie Methyl, Ethyl, Propyl oder Butyl sein, wobei der Methylrest besonders bevorzugt ist. Auch alicyclische Reste, bei denen R¹ und R² bzw. R³ und R⁴ zusammen eine Tetramethylen- oder eine Pentamethylengruppe darstellen, kommen in Betracht.

Der Molekülteil A¹ dient besonders der Anpassung der chemischen und physikalischen Eigenschaften der Verbindungen an die unterschiedlichen Verwendungszwecke. Durch Variation des Molekülteils A¹ kann z.B. die Löslichkeit in verschiedenen organischen Materialien, die Flüchtigkeit sowie die Verträglichkeit mit anderen Hilfsstoffen beeinflußt werden.

A¹ ist hierbei ein organischer Rest ausgewählt aus der Gruppe bestehend aus:
- C₁-C₂₂-Alkyl wie Methyl, Ethyl, n- und i-Propyl, n- und i-Butyl, n- und i-Pentyl, Hexyl, Octyl, Decyl, Dodecyl, Octadecyl, Pivalyl, 3,3-Dimethylbut-2-yl, Neopentyl, 4-Methylpent-2-yl und 2-Ethylhexyl,
- C₃-C₂₂-Alkenyl wie Allyl, Butenyl, Pentenyl und Oleyl,
- C₃-C₁₂-Cycloalkyl wie Cyclopropyl, Cyclobutyl, Cyclopentyl, Cyclohexyl, Methylcyclohexyl, Cycloheptyl, Cyclooctyl, Cyclododecyl und Bicycloheptyl, sowie vor allem Cyclopentyl und Cyclohexyl,
- durch Cyan, Hydroxy oder Carboalkoxy substituiertes C₂-C₂₂-Alkyl wie Cyanmethyl, Hydroxyethyl, Hydroxypropyl, Hydroxybutyl, Carbomethoxyethyl und Carboethoxyethyl,
- durch Ethersauerstoff oder Stickstoff unterbrochenes oder durch Hydroxy substituiertes C₄-C₂₂-Alkyl wie -(CH₂)₃N(CH₃)₂, -(CH₂)₃N(C₂H₅)₂, -(CH₂)₃-OCH₃, -(CH₂)₃-O-CH(CH₃)₂, -(CH₂)₂O(CH₂)₂-OH, -CH₂-(CH₂)₂-CH₂-N(CH₂)₃, -(CH₂)₂-N[CH(CH₃)₂]₂, -(CH₂)₂-N(C₂H₅)₂, -(CH₂)₂N(CH₃)₂, -(CH₂)₂OCH₃ und -(CH₂)₂OCH₂CH₃,
- substituierte C₇-C₂₂-Phenyl- und C₁₃-C₂₂-Diphenylalkylreste wie Benzyl, die isomeren Methoxybenzyle, Methylbenzyle, Ethylbenzyle, Isopropylbenzyle, Trimethylbenzyle, Fluorbenzyle, Chlorbenzyle, Methylendioxybenzyle, Phenylethyle, Phenylpropyle und Phenylbutyle, Dimethylaminobenzyle, Diphenylmethyl und 1,3-Diphenylprop-2-yl,
- Arylreste wie Phenyl, Tolyl und durch Carbo-C₁-C₄-alkoxy substituiertes Phenyl,
- Heterocyclen tragendes C₁-C₂₂-Alkyl wie 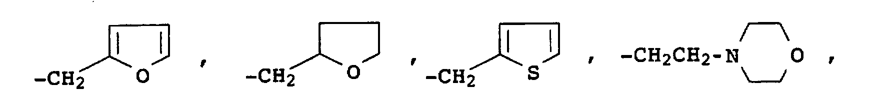 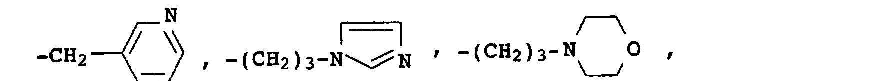 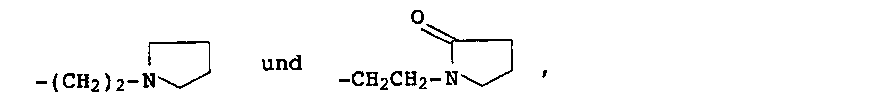
- phosphorhaltige Gruppen wie Phosphoramide, Phosphinsäurederivate, Phosphorsäurederivate mit Alkylresten oder stickstoff- und/oder sauerstoffhaltigen organischen Resten aliphatischer, aromatischer oder heterocyclischer Art.

Bevorzugte Verbindungen Ia sind solche, in denen A¹ seinerseits noch weitere Reste IIa trägt. Dadurch erhalten die Verbindungen einerseits eine Molmasse, die ihre Flüchtigkeit stark herabsetzt, und andererseits ist durch das Vorhandensein zweier aktiver Stabilisatorgruppen die stabilisierende Wirkung der Verbindungen erhöht. Unter den Verbindungen der Formel Ia erfüllen besonders die Verbindungen der Formel Ib diese Eigenschaften.

Die Verbindungen Ib können neben den Molekülteilen IIa auch Molekülteile IIb enthalten, in denen der Rest R⁷ Wasserstoff, die Hydroxylgruppe sowie C- oder O-organische Reste sein können. Als solche Reste können vor allem Formyl, O-Alkyl, O-Aryl, O-Hydrocarbyl, O-Carbamoyl, Cyanmethyl oder substituiertes Alkenyl dienen. Für die Reste R¹ bis R⁴ gelten dabei die gleichen Möglichkeiten und Präferenzen wie sie für die Molekülteile IIa angegeben wurden.

Die Molekülteile A² stehen hierbei für n-wertige organische Reste ausgewählt aus der Gruppe bestehend aus:
- C₂-C₂₂-Alkylen und C₅-C₂₂-Cycloalkylen wie -(CH₂)ₚ-CH₂-(mit p = 1 bis 21) 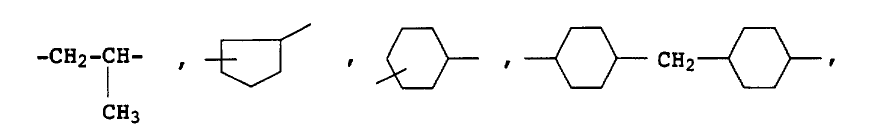 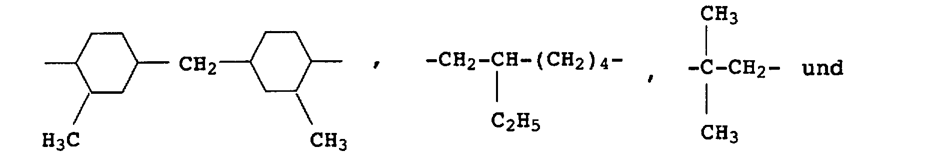 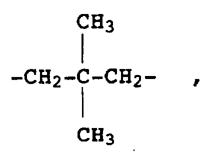
- C₈-C₁₄-Phenylalkylen und Phenylen wie 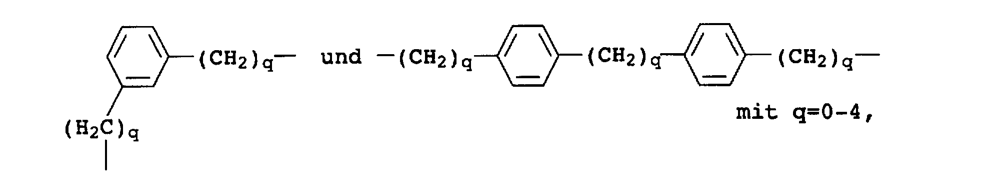
- durch Ethersauerstoff, Stickstoff oder Heterocyclen unterbrochenes Alkylen wie
   -(CH₂)₃O(CH₂)₄O(CH₂)₃-, -(CH₂)₃O(CH₂)₂O(CH₂)₂O(CH₂)₃-, 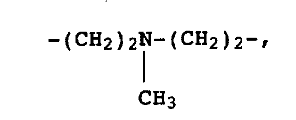 -(C₃H₆O)ᵣ-C₃H₆- mit r=1 bis 33,
   -(CH₂)₃N-(CH₂)₃-, 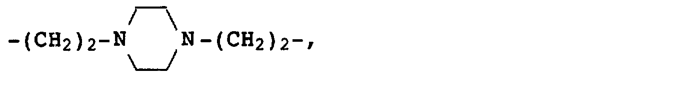 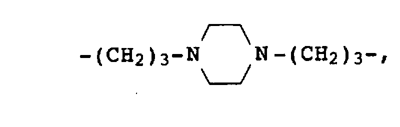 -(CH₂)₃O(CH₂)₂O(CH₂)₃-, 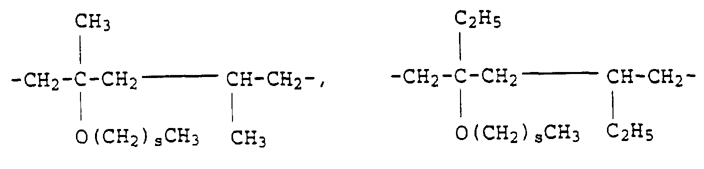 oder 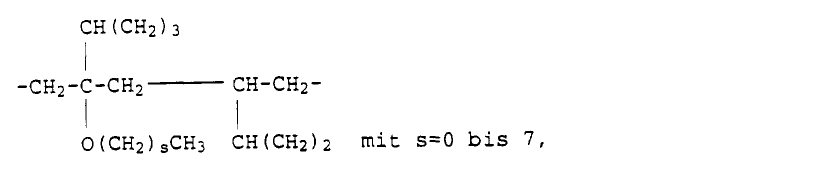
- kohlenstoff-, sauerstoff- und/oder stickstoffhaltige Brücken mit Phosphor als Heteroatom wie
   -(CH₂)-O-P(O)Ph-O-(CH₂)₂-, -(CH₂)₂-O-P(O)OPh-O-(CH₂)₂-,

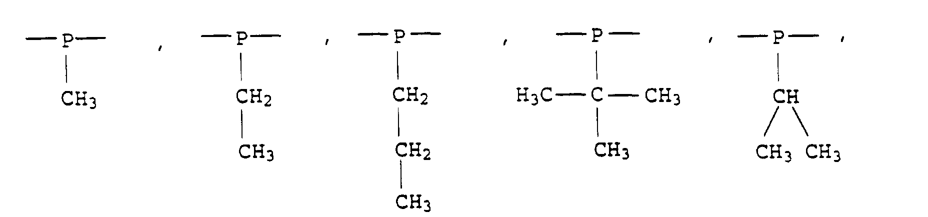

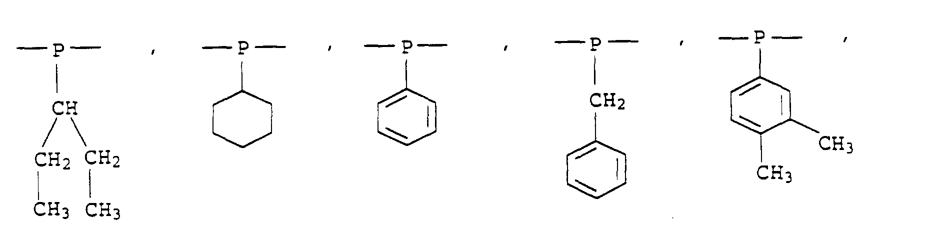

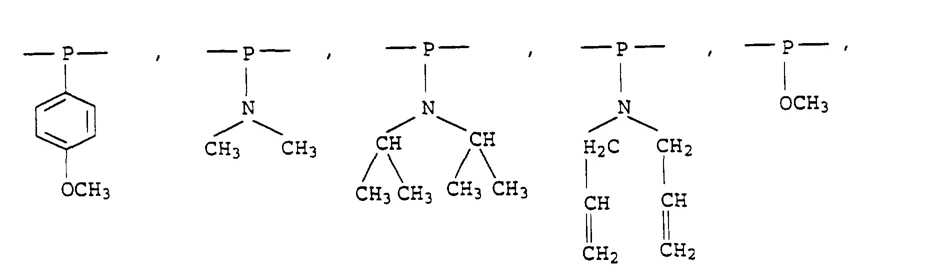

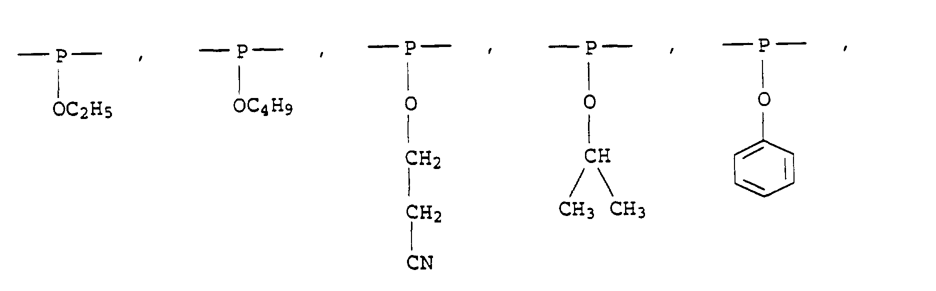

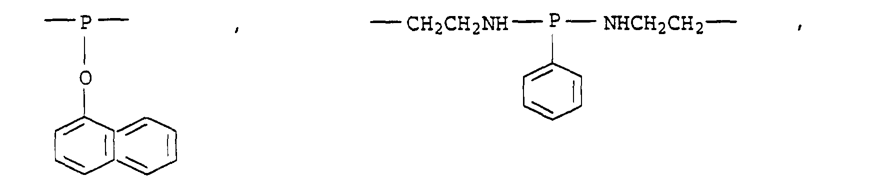

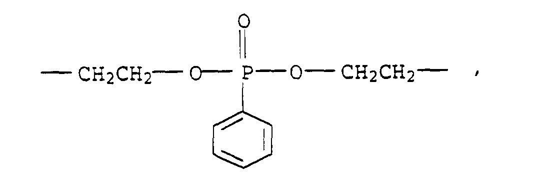

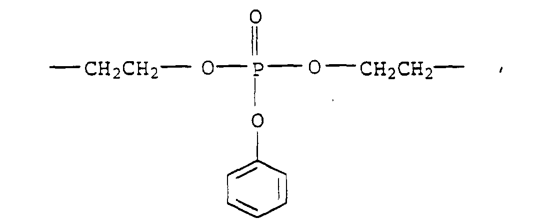

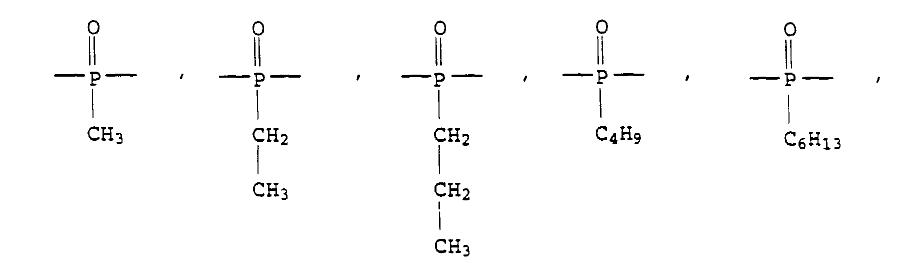

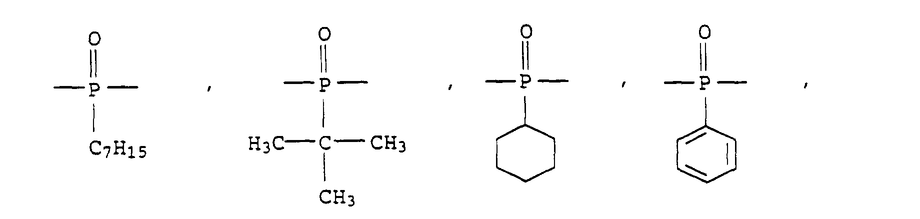

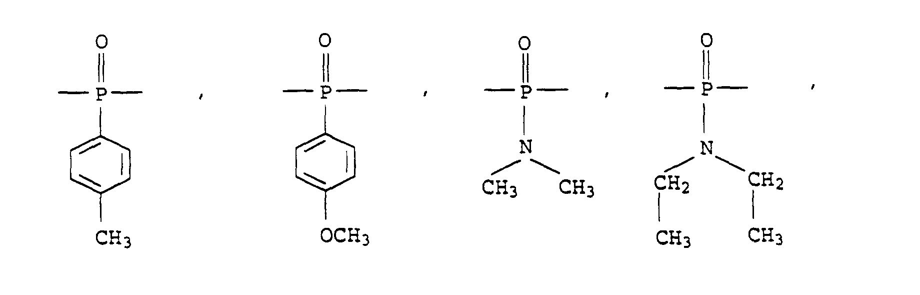

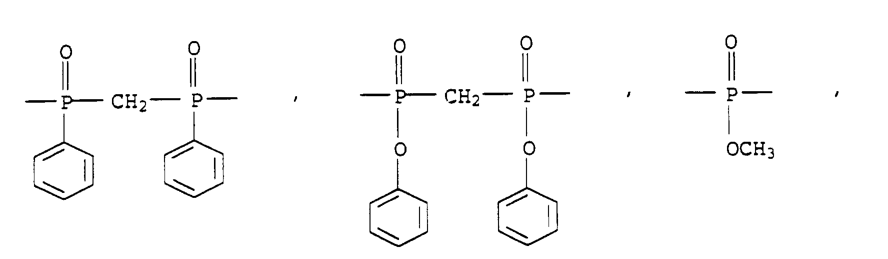

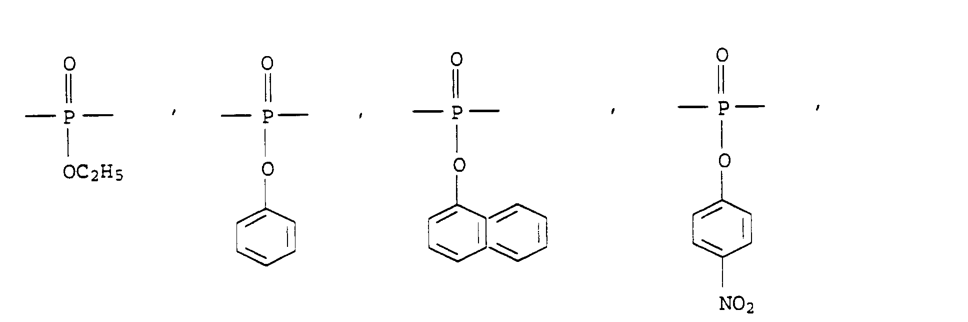

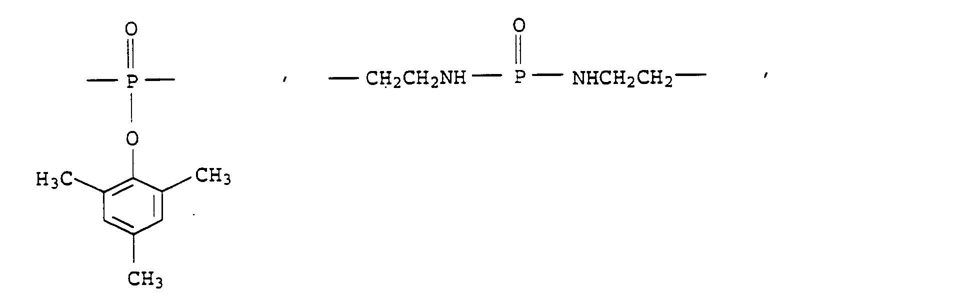

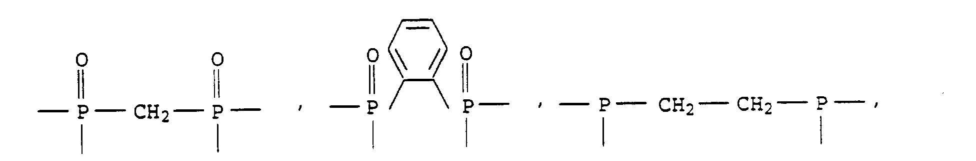

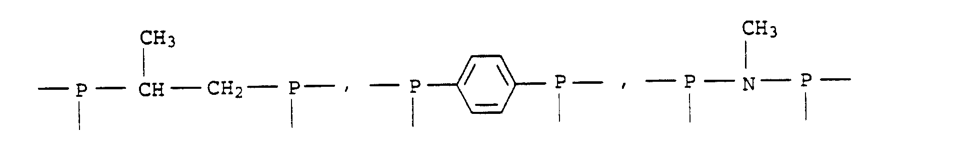

Besonders bevorzugte Verbindungen sind Verbindungen der Formel Ib, in denen alle Molekülteile B² N-Oxylpiperidinreste sind und der Molekülteil A² einen kurzkettigen Alkylenrest mit 2 bis 8 Kohlenstoffatomen darstellt, wie -CH₂-CH₂-, -(CH₂)₄-, -(CH₂)₆- oder - (CH₂)₈-.

Die erfindungsgemäßen Verbindungen können durch Oxidation der entsprechenden Piperidinverbindungen mit Wasserstoffperoxid hergestellt werden. Die eingesetzten Piperidinverbindungen und deren Herstellung sind z.B. in der EP-A 0 316 582 beschrieben oder sind in bekannter Weise herstellbar. Die Oxidationsreaktion wird bevorzugt unter Zusatz organischer Lösungsmittel durchgeführt.

Als organische Lösungsmittel werden bevorzugt solche verwendet, die zumindest teilweise mit Wasser mischbar sind, also z.B. polare protische Lösungsmittel wie Alkohole, besonders Methanol, Ethanol, Propanol, n-Butanol und iso-Butanol und polare aprotische Lösungsmittel wie Dimethylformamid, Dimethylacetamid, N-Methylpyrrolidon und Tetrahydrothiophendioxid.

Die Oxidation wird bevorzugt bei einem pH-Wert zwischen 6,5 und 11 durchgeführt, besonders bevorzugt zwischen 7,0 und 8,5, ganz besonders bevorzugt zwischen 7,5 und 8,0, wobei der pH-Wert durch Anwesenheit von puffernden Verbindungen wie Natriumcarbonat, Natriumhydrogencarbonat, Kalium- oder Natrium-mono- oder dihydrogenphosphat, Natrium- oder Kaliumhydrogensulfat oder auch organischen Säuren wie Essigsäure, Ameisensäure, Cyanessigsäure, Malonsäure oder Milchsäure auf den gewünschten Wert eingestellt und konstant gehalten werden kann. Zur Einstellung des pH-Wertes eignen sich weiterhin anorganische Säuren wie Salzsäure, Schwefelsäure oder Phosphorsäure sowie anorganische Basen wie Natriumhydroxid, Lithiumhydroxid oder, besonders bevorzugt, Kaliumhydroxid, welches als wäßrige oder alkoholische Lösung eingesetzt werden kann.

Darüber hinaus ist die Mitverwendung katalytischer Mengen von Oxiden, Hydroxiden oder Salzen des Magnesiums, Calciums oder Zinks vorteilhaft, wobei als Anionen Clorid, Bromid, Sulfat und Phosphat in Betracht kommen. Gute Ergebnisse erzielt man z.B. mit Magnesiumsulfat. Auch Borsäure, deren Salze und Hydrate dieser Salze lassen sich vorteilhaft als Katalysatoren verwenden. Die Konzentration der Salze liegt bei 0,01 bis 10 mol-%, bevorzugt bei 0,1 mol-%, bezogen auf die Menge des Acylaminopiperidins.

Die Oxidation wird bevorzugt bei 40 bis 100°C, besonders bei 60 bis 80°C durchgeführt. Nach Reaktionszeiten von 0,5 bis etwa 24 Stunden kühlt man das Reaktionsgemisch zweckmäßigerweise auf Raumtemperatur ab, versetzt es mit Wasser und trennt die hierbei anfallenden Reaktionsprodukte in an sich bekannter Weise als Feststoffe ab. Besonders bei Reaktionen, die in Gegenwart leicht flüchtiger Lösungsmittel, beispielsweise Methanol, Ethanol oder Isopropanol, durchgeführt werden, kann zur Aufarbeitung das Lösungsmittel mit oder ohne Zusatz von Wasser durch Destillation entfernt werden. Die Oxidationsbedingungen sind so mild und selektiv, daß es nicht zur Abspaltung empfindlicher Gruppen, beispielsweise des Formylrestes, kommt. Nach Beendigung der Oxidation ist es vorteilhaft, überschüssiges Wasserstoffperoxid zu zersetzen. Dies kann z.B. durch Behandlung des Reaktionsgemisches mit Eisen- oder Mangansalzen, bevorzugt mit Eisen-II-Sulfat, bei leicht alkalischem pH-Wert, z.B. bei pH 9-10, geschehen.

Die Oxidationsreaktion muß dabei nicht unbedingt vollständig ablaufen. Auch die teilweise oxidierten Piperidinverbindungen zeigen bereits gute Wirksamkeit.

Die erfindungsgemäßen 4-Acylaminopiperidin-N-oxyle eignen sich zur Stabilisierung organischer Materialien und schützen diese vor der schädigenden Wirkung von Licht und Wärme. Zu den organischen Materialien, die durch die Verbindungen stabilisiert werden können, gehören Kunststoffe aller Art, z.B. Polypropylen, Polyethylen, Acrylnitril-Butadien-Styrol-Copolymere, Polyamide, Polyurethane sowie auch pigmenthaltige Polyolefine. Auch Fette, Öle und Lacke lassen sich mit den erfindungsgemäßen Verbindungen stabilisieren.

Besonderen Vorteil bieten die Stabilisatoren Ia und Ib zur Stabilisierung von radikalisch polymerisierenden Monomeren wie den Estern und Amiden der Acrylsäure und Methacrylsäure sowie dieser Säuren selbst, Acrylnitril, Methacrylnitril, Vinylchlorid und Styrol.

Besonders für die Stabilisierung während der Lagerung und bei der Destillation können die erfindungsgemäßen Verbindungen vorteilhaft eingesetzt werden. Besondere Bedeutung haben die erfindungsgemäßen Verbindungen Ia und Ib im Falle der Destillation des polymerisationsempfindlichen Styrols.

Die erfindungsgemäßen Verbindungen Ia und Ib weisen bereits für sich eine sehr gute stabilisierende Wirkung auf. Diese Wirkung kann oft noch gesteigert werden durch die Kombination mit aromatischen Nitro- oder Nitrosoverbindungen oder mit substituierten Phenolen. Als aromatische Nitroverbindungen können beispielsweise
1,3-Dinitrobenzol,
1,4-Dinitrobenzol,
2,6-Dinitro-4-methylphenol,
2-Nitro-4-methylphenol,
2,4,6-Trinitrophenol,
2,4-Dinitro-1-naphthol,
2,4-Dinitro-6-methylphenol,
2,4-Dinitrochlorbenzol,
2,4-Dinitrophenol,
2,4-Dinitro-6-sec-butylphenol,
4-Cyano-2-nitrophenol,
3-Iodo-4-cyano-5-nitrophenol,
besonders bevorzugt 2,6-Dinitro-4-methylphenol,
2-Nitro-4-methylphenol,
2,4-Dinitro-6-sec-butylphenol bzw.
2,4-Dinitro-6-methylphenol
verwendet werden.

Als aromatische Nitrosoverbindungen kommen z.B.
p-Nitrosophenol,
p-Nitroso-o-kresol und
p-Nitroso-N,N-diethylanilin
in Betracht.

Als substituierte Phenole kommen z.B. in Betracht:
4-tert-Butylbrenzcatechin,
Methoxyhydrochinon,
2,6-Di-tert-butyl-4-methylphenol,
n-Octadecyl-β-(3,5-di-tert-butyl-4-hydroxyphenyl)-propionat,
1,1,3-Tris-(2-methyl-4-hydroxy-5-tert-butylphenyl)-butan,
1,3,5-Trimethyl-2,4,6-tris-(3,5-di-tert-butyl-4-hydroxybenzyl)-benzol,
1,3,5-Tris-(3,5-di-tert-butyl-4-hydroxybenzyl)-isocyanurat,
1,3,5-Tris-[β-(3,5-di-tert-butyl-4-hydroxyphenyl)-propionyloxyethyl]-isocyanurat,
1,3,5-Tris-(2,6-dimethyl-3-hydroxy-4-tert-butylbenzyl)-isocyanurat und
Pentaerythrit-tetrakis-[β-(3,5-di-tert-butyl-4-hydroxy-phenyl)-propionat].

Die 4-Acylaminopiperidin-N-oxyle Ia und Ib können gewünschtenfalls auch in beliebiger Kombination mit anderen N-Oxylen eingesetzt werden, z.B. mit
Di-tert.-butyl-nitroxyl,
1-Oxyl-2,2,6,6-tetramethylpiperidin,
1-Oxyl-2,2,6,6-tetramethylpiperidin-4-ol,
1-Oxyl-2,2,6,6-tetramethylpiperidin-4-on,
1-Oxyl-2,2,6,6-tetramethylpiperidin-4-yl-acetat,
1-Oxyl-2,2,6,6-tetramethylpiperidin-4-yl-2-ethylhexanoat,
1-Oxyl-2,2,6,6-tetramethylpiperidin-4-yl-stearat,
1-Oxyl-2,2,6,6-tetramethylpiperidin-4-yl-benzoat,
1-Oxyl-2,2,6,6-tetramethylpiperidin-4-yl-(4-tert-butyl)benzoat,
Bis(1-oxyl-2,2,6,6-tetramethylpiperidin-4-yl)-succinat,
Bis(1-oxyl-2,2,6,6-tetramethylpiperidin-4-yl)-adipat,
Bis(1-oxyl-2,2,6,6-tetramethylpiperidin-4-yl)-sebacat,
Bis(1-oxyl-2,2,6,6-tetramethylpiperidin-4-yl)-n-butylmalonat,
Bis(1-oxyl-2,2,6,6-tetramethylpiperidin-4-yl)-phthalat,
Bis(1-oxyl-2,2,6,6-tetramethylpiperidin-4-yl)-isophthalat,
Bis(1-oxyl-2,2,6,6-tetramethylpiperidin-4-yl)-terephthalat,
Bis(1-oxyl-2,2,6,6-tetramethylpiperidin-4-yl)-hexahydroterephthalat,
N,N'-Bis(1-oxyl-2,2,6,6-tetramethylpiperidin-4-yl)-adipinamid,
N-(1-Oxyl-2,2,6,6-tecramethylpiperidin-4-yl)-caprolactam,
N-(1-Oxyl-2,2,6,6-tetramethylpiperidin-4-yl)-dodecylsuccinimid,
2,4,6-Tris-[N-butyl-N-(1-oxyl-2,2,6,6-tetramethylpiperidin-4-yl]-s-triazin und
4,4'-Ethylenbis(1-oxyl-2,2,6,6-tetramethylpiperazin-3-on).

Auch die Kombination mit sterisch gehinderten Aminen wie 2,2,6,6-Tetramethylpiperidinderivaten ist vorteilhaft. Dazu gehören auch die nicht oxidierten Ausgangsverbindungen für die erfindungsgemäßen Verbindungen.

In allen Fällen können bis zu 50 Gew.-% von Ia oder Ib durch andere Oxylverbindungen ersetzt werden.

Für die Stabilisierungszwecke verwendet man die erfindungsgemäßen Verbindungen vorzugsweise in folgenden Konzentrationen:
- Im Falle der Stabilisierung von Kunststoffen:
   Ia bzw. Ib allein: 0,01 bis 5, vorzugsweise 0,02 bis 1 Gew.-%, bezogen auf die Menge des Kunststoffs.
- Im Falle der Stabilisierung von Fetten, Ölen und Lacken:
   0,01 bis 5, vorzugsweise 0,02 bis 1 Gew.-%.
- Im Falle der Lagerung von radikalisch polymerisierenden Monomeren:
   0,0002 bis 0,1, vorzugsweise 0,0005 bis 0,01 Gew.-%.
- Im Falle der Destillation von radikalisch polymerisierenden Monomeren:
   0,0005 bis 0,5, vorzugsweise 0,005 bis 0,05 Gew.-%.
   Ia bzw. Ib in Verbindung mit einer aromatischen Nitro- oder Nitrosoverbindung oder einem substituierten Phenol als Costabilisator: 0,0005 bis 0,5, vorzugsweise 0,005 bis 0,05 Gew.-% Ia bzw. Ib plus 0,001 bis 0,5 Gew.-% Costabilisator.

### Beispiele

### Beispiel 1

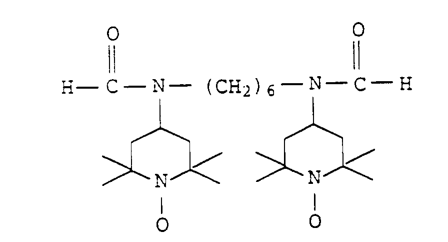

Eine Suspension aus 540 g (1,37 mol) N,N'-Bis-[2,2,6,6-tetramethylpiperidin-4-yl]-N,N'-bis-formyl-1,6-diaminohexan, 800 ml Wasser, 150 ml Isobutanol und 200 mg MgSO₄ wurde bei 70°C im Laufe von 2 h mit 600 ml einer 30 gew.-%igen Lösung von Wasserstoffperoxid (19,6 mol) versetzt und anschließend noch 16 h bei dieser Temperatur gehalten. Danach wurde die Mischung auf Raumtemperatur abgekühlt und das ausgefallene Produkt wie üblich isoliert. Das Produkt enthält neben dem Di-N-Oxyl als Nebenprodukt die Mono-N-Oxylverbindung.
Ausbeute: 85 %, Schmelzpunkt: 169 bis 170°C.

Analog zu Beispiel 1, jedoch mit Methanol statt Wasser/Isobutanol als Lösungsmittel, wurden die folgenden Beispielverbindungen hergestellt:

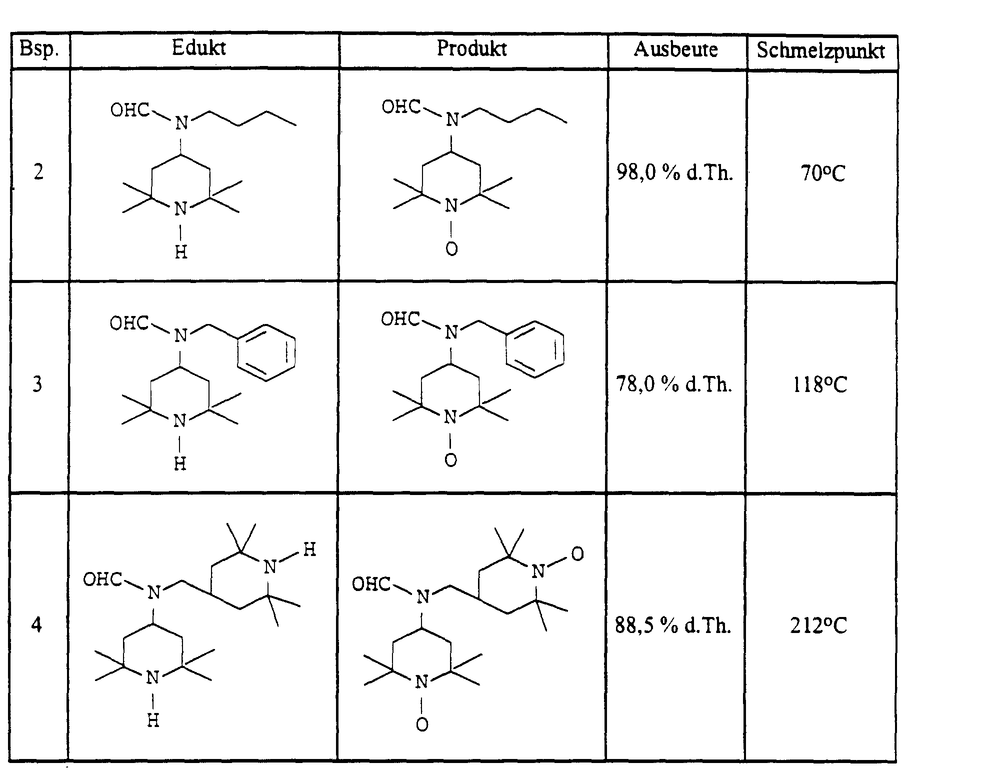

### Beispiel 5

Stabilisierende Wirkung der Verbindung gemäß Beispiel 1 in Styrol:

Der Stabilisator gemäß Beispiel 1 sowie zum Vergleich verschiedene herkömmliche Stabilisatoren wurden in einer Konzentration von 120 ppm in Styrol gelöst. 500 ml dieser Lösung wurden in einem Reaktionsgefäß unter Stickstoff auf 110°C erhitzt. In diese temperierte Styrollösung wurden 250 g einer identischen Lösung pro Stunde kontinuierlich zudosiert und die gleiche Menge kontinuierlich entnommen. Im Auslaß wurde der Gleichgewichtspolymergehalt gemessen. Folgende Ergebnisse wurden festgestellt:

| Stabilisator | Polymergehalt in % |
|---|---|
| erfindungsgemäß: | |
| Verbindung gemäß Beispiel 1 | 0,10 |

| zum Vergleich: | |
|---|---|
| Bis(1-oxyl-2,2,6,6-tetramethylpiperidin-4-yl)sebacat | 0,24 |
| p-Nitrosophenol | 0,18 |
| p-Nitroso-o-kresol | 0,30 |
| ohne Stabilisator: | >5,00 * |

| | |
|---|---|
| * Abbruch vor Einstellung des Gleichgewichtes | |

### Beispiel 6

Stabilisierende Wirkung der Verbindung gemäß Beispiel 1 in Kombination mit einem Costabilisator:

Der Stabilisator gemäß Beispiel 1 sowie im Vergleich dazu ein herkömmlicher Stabilisator wurden in einer Konzentration von 120 ppm in Styrol gelöst. Zusätzlich wurde 2,4-Dinitro-sec-butylphenol als Costabilisator in einer Konzentration von 240 ppm in diesem Styrol gelöst. Die Lösungen wurden einem Versuch wie in Beispiel 5 unterzogen und wiederum der Polymergehalt im Auslaß gemessen. Folgende Ergebnisse wurden festgestellt:

| Stabilisator | Polymergehalt in % |
|---|---|
| erfindungsgemäß: | |
| Verbindung gemäß Beispiel 1 + 2,4-Dinitro-sec-butylphenol | 0,04 |

| zum Vergleich: | |
|---|---|
| Bis(1-oxyl-2,2,6,6-tetramethylpiperidin-4-yl)sebacat + 2,4-Dinitro-sec-butylphenol | 0,07 |

### Beispiel 7

Stabilisierende Wirkung der Verbindung gemäß Beispiel 1 in Acrylsäure:

Acrylsäure wurde unter Zusatz von 50 ppm des Stabilisators gemäß Beispiel 1 sowie im Vergleich dazu verschiedener herkömmlicher Stabilisatoren in einer Ampulle eingeschmolzen und bei 80°C thermostatisiert. Es wurde die Induktionsperiode bis zum Beginn der Polymerisation gemessen. Folgende Ergebnisse wurden festgestellt:

| Stabilisator | Induktionsperiode [h] |
|---|---|
| erfindungsgemäß: | |
| Verbindung gemäß Beispiel 1 | 502 |

| zum Vergleich: | |
|---|---|
| Bis(1-oxyl-2,2,6,6-tetramethyl-piperidin-4-yl)sebacat | 470 |
| Phenothiazin | 170 |
| ohne Stabilisator: | 1 |

## Patentansprüche

1. 4-Formylaminopiperidin-N-oxyle der allgemeinen Formal Ia
A¹B¹ (Ia)
in der A¹ für einen organischen Rest ausgewählt aus der Gruppe bestehend aus
C₁-C₂₂-Alkyl,
C₃-C₂₂-Alkenyl,
C₃-C₁₂-Cycloalkyl,
durch Cyan, Hydroxy oder Carboalkoxy substituiertes C₂-C₂₂-Alkyl,
durch Ethersauerstoff oder Stickstoff unterbrochenes oder durch Hydroxy substituiertes C₄-C₂₂-Alkyl,
substituierte C₇-C₂₂-Phenyl- und C₁₃-C₂₂-Diphenylalkylreste,
Arylreste,
Heterocyclen tragendes C₁-C₂₂-Alkyl und
phosphorhaltige Gruppen
und B¹ für einen Rest der allgemeinen Formel IIa steht 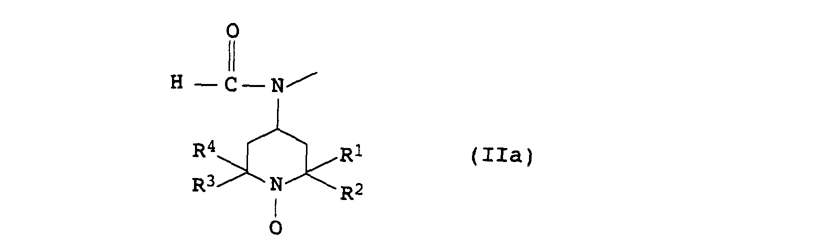
in welcher die Substituenten folgende Bedeutung haben:
R¹-R⁴ C₁-C₄-Alkyl, wobei R¹ und R² sowie R³ und R⁴ auch zu einem 5- oder 6-gliedrigen Ring verbunden sein können,

2. 4-Formylaminopiperidin-N-oxyle der allgemeinen Formel Ib
A²(B²)ₙ (Ib)
in der A² für einen n-wertigen organischen Rest ausgewählt aus der Gruppe bestehend aus
C₂-C₂₂-Alkylen und C₅-C₂₂-Cycloalkylen,
C₈-C₁₄-Phenylalkylen und Phenylen,
durch Ethersauerstoff, Stickstoff oder Heterocyclen unterbrochenes Alkylen und
kohlenstoff-, sauerstoff- und/oder stickstoffhaltige Brücken mit Phosphor als Heteroatom steht,
n den Wert 2 bis 4 hat und B² mindestens einen der Reste IIa und die übrigen Reste B² gleiche oder verschiedene Reste der Formel IIb 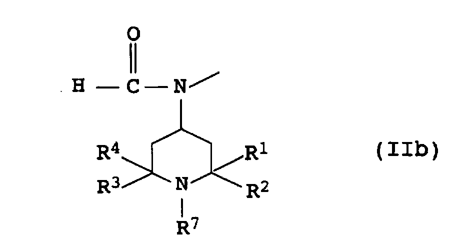
bedeuten, wobei R⁷ Wasserstoff, die Hydroxygruppe oder Formyl, O-Alkyl, O-Aryl, O-Hydrocarbyl, O-Carbamoyl, Cyanmethyl oder substituiertes Alkenyl bedeutet.

3. 4-Formylaminopiperidin-N-oxyle nach den Ansprüchen 1 oder 2, in denen R¹ bis R⁴ für Methylgruppen stehen.

4. 4-Formylaminopiperidin-N-oxyle nach den Ansprüchen 2 oder 3, in denen A² eine α,ω-Alkylengruppe mit 2 bis 8 Kohlenstoffatomen bedeutet.

5. N,N'-Bis(1-oxyl-2,2,6,6-tetramethylpiperidin-4-yl)-N,N'-bis-formyl-1,6-diaminohexan.

6. Verfahren zur Herstellung der 4-Formylaminopiperidin-N-oxyle gemäß den Ansprüchen 1 bis 5, **dadurch gekennzeichnet, daß** man die entsprechenden N-unsubstituierten Piperidine mit Wasserstoffperoxid oxidiert.

7. Verwendung der 4-Formylaminopiperidin-N-oxyle gemäß den Ansprüchen 1 bis 5 zur Stabilisierung organischer Materialien gegen die schädigende Wirkung von Radikalen.

8. Verwendung der 4-Formylaminopiperidin-N-oxyle nach Anspruch 7 zur Stabilisierung von radikalisch polymerisierenden Monomeren.

9. Verwendung der 4-Formylaminopiperidin-N-oxyle nach Anspruch 8 zur Stabilisierung von Styrol.

10. Verfahren zur Reindarstellung von radikalisch polymerisierenden Monomeren durch Destillation der verunreinigten Monomeren, **dadurch gekennzeichnet, daß** man die Destillation in Gegenwart eines 4-Formylaminopiperidin-N-oxyls gemäß den Ansprüchen 1 bis 5 vornimmt.

11. Verfahren nach Anspruch 10, **dadurch gekennzeichnet, daß** man es auf die Reindarstellung von Styrol anwendet.

12. Verwendung der 4-Formylaminopiperidin-N-Oxyle nach den Ansprüchen 7 bis 10 zusammen mit aromatischen Nitro- oder Nitrosoverbindungen oder substituierten Phenolen oder Mischungen dieser Verbindungen.

13. Mischungen aus 4-Formylaminopiperidin-N-Oxylen gemäß den Ansprüchen 1 bis 5 und aromatischen Nitro- oder Nitrosoverbindungen oder substituierten Phenolen.

14. Kunststoffe, Lacke, Öle, Fette und radikalisch polymerisierende Monomere, enthaltend ein 4-Formylaminopiperidin-N-oxyl gemäß den Ansprüchen 1 bis 5.

## Claims

1. A 4-formylaminopiperidin-N-oxyl of the formula Ia
A¹B¹ (Ia)
A¹ is an organic radical selected from the group consisting of
C₁-C₂₂-alkyl,
C₃-C₂₂-alkenyl,
C₃-C₁₂-cycloalkyl,
cyano, hydroxyl or carboalkoxy-substituted C₂-C₂₂-alkyl,
C₄-C₂₂-alkyl interrupted by ether oxygen or nitrogen or substituted by hydroxyl,
substituted C₇-C₂₂-phenyl and C₁₃-C₂₂-diphenylalkyl radicals,
aryl radicals,
C₁-C₂₂-alkyl carrying heterocycles and
phosphorus-containing groups
and B¹ is a radical of the formula IIa 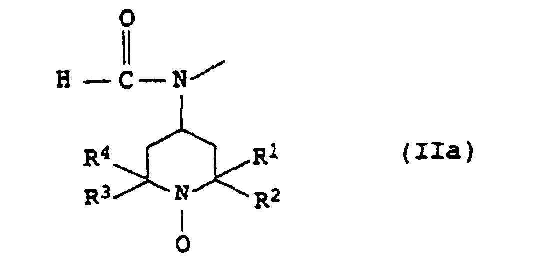 where
R¹-R⁴ are each C₁-C₄-alkyl, it also being possible for R¹ and R² on the one hand and R³ and R⁴ on the other hand to be linked to form a 5- or 6-membered ring,
excluding the compound 4-[N-formyl-N-(3-hydroxypropyl)amino]-2,2,6,6-tetramethylpiperidin-1-oxyl.

2. A 4-formylaminopiperidin-N-oxyl of the formula Ib
A²(B²)ₙ (Ib)
where A² is an n-valent organic radical selected from the group consisting of
C₂-C₂₂-alkylene and C₅-C₂₂-cycloalkylene,
C₈-C₁₄-phenylalkylene and phenylene,
alkylene interrupted by ether oxygen, nitrogen or heterocycles and
carbon-, oxygen- and/or nitrogen-containing bridges having phosphorus as a hetero atom,
n is from 2 to 4 and B² is at least one of the radicals IIa and the remaining radicals B² are identical or different radicals of the formula IIb 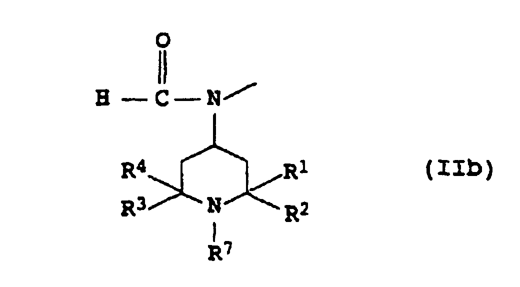 where R⁷ is hydrogen, hydroxyl, formyl, O-alkyl, O-aryl, O-hydrocarbyl, O-carbamoyl, cyanomethyl or substituted alkenyl.

3. A 4-formylaminopiperidin-N-oxyl as claimed in claim 1 or 2, in which R¹ to R⁴ are each methyl.

4. A 4-formylaminopiperidin-N-oxyl as claimed in claim 2 or 3, in which A² is an α,ω-alkylene group of 2 to 8 carbon atoms.

5. N,N'-Bis(1-oxyl-2,2,6,6-tetramethylpiperidin-4-yl)-N,N'-bisformyl-1,6-diaminohexane.

6. A process for the preparation of a 4-formylaminopiperidin-N-oxyl as claimed in any of claims 1 to 5, wherein the corresponding N-unsubstituted piperidine is oxidized with hydrogen peroxide.

7. The use of a 4-formylaminopiperidin-N-oxyl as claimed in any of claims 1 to 5 for stabilizing organic materials against the harmful effect of free radicals.

8. The use of a 4-formylaminopiperidin-N-oxyl as claimed in claim 7 for stabilizing monomers capable of free radical polymerization.

9. The use of a 4-formylaminopiperidin-N-oxyl as claimed in claim 8 for stabilizing styrene.

10. A process for the purification of monomers capable of free radical polymerization by distillation of the contaminated monomers, wherein the distillation is carried out in the presence of a 4-formylaminopiperidin-N-oxyl as claimed in any of claims 1 to 5.

11. A process as claimed in claim 10, which is applied to the purification of styrene.

12. The use of a 4-formylaminopiperidin-N-oxyl as claimed in any of claims 7 to 10 together with aromatic nitro or nitroso compounds or substituted phenols or mixtures of these compounds.

13. A mixture of a 4-formylaminopiperidin-N-oxyl as claimed in any of claims 1 to 5 and aromatic nitro or nitroso compounds or substituted phenols.

14. A plastic, finish, oil or fat or a monomer capable of free radical polymerization, containing a 4-formylaminopiperidin-N-oxyl as claimed in any of claims 1 to 5.

## Revendications

1. N-oxyles de 4-formylaminopipéridine de formule générale Ia
A¹B¹ (Ia)
dans laquelle A¹ est un radical organique choisi dans l'ensemble comprenant
les groupes alkyle en C₁-C₂₂,
les groupes alcényle en C₃-C₂₂,
les groupes cycloalkyle en C₃-C₁₂,
les groupes alkyle en C₂-C₂₂ à substitution cyano, hydroxy ou carbalcoxy,
les groupes alkyle en C₄-C₂₂ interrompus par un oxygène en fonction éther ou un azote, ou hydroxylés,
les radicaux phénylalkyle en C₇-C₂₂ ou diphénylalkyle en C₁₃-C₂₂ substitués,
les groupes aryle,
les groupes alkyle en C₁-C₂₂ portant des hétérocycles, et
les groupes contenant du phosphore,
et B¹ est un radical de formule générale IIa 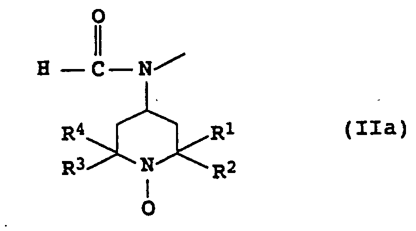
dans laquelle les substituants ont les significations suivantes :
R¹-R⁴ sont des groupes alkyle en C₁-C₄, où R¹ et R², ainsi que R³ et R⁴, peuvent aussi être liés pour former un noyau à 5 ou 6 chaînons, à l'exception du composé 1-oxyle de 4-[N-formyl-N(3-hydroxypropyl)amino]-2,2,6,6-tétraméthylpipéridine.

2. N-oxyles de 4-formylaminopipéridine de formule générale Ib
A²(B²)ₙ (Ib)
dans laquelle A² est un radical organique n-valent choisi dans l'ensemble comprenant :
les enchaînements alkylène en C₂-C₂₂ ou cycloalkylène en C₅-C₂₂,
les enchaînements phénylalkylène en C₈-C₁₄ et phénylène,
les enchaînements alkylène interrompus par un oxygène en fonction éther, un azote ou des hétérocycles, et
les ponts carbonés, oxygénés et/ou azotés, avec du phosphore en tant qu'hétéroatome,
n a une valeur de 2 à 4, et B² est au moins l'un des radicaux IIa, et les autres radicaux B² sont des radicaux identiques ou différents de formule IIb 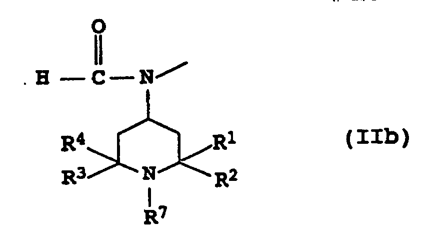
dans laquelle R⁷ est un atome d'hydrogène, le groupe hydroxy, ou un groupe formyle, O-alkyle, O-aryle, O-hydrocarbyle, O-carbamoyle, cyanométhyle ou alcényle substitué.

3. N-oxyles de 4-formylaminopipéridine selon les revendications 1 ou 2, dans lesquels R¹ à R⁴ sont des groupes méthyle.

4. N-oxyles de 4-formylaminopipéridine selon les revendications 2 ou 3, dans lesquels A² est un groupe α,ω-alkylène ayant de 2 à 8 atomes de carbone.

5. N,N'-bis(1-oxyle-2,2,6,6-tétraméthylpipéridine-4-yl)-N,N'-bis-formyl-1,6-diaminohexane.

6. Procédé de préparation des N-oxyles de 4-formylaminopipéridine selon les revendications 1 à 5, **caractérisé en ce qu'**on oxyde avec du peroxyde d'hydrogène les pipéridines N-non-substituées correspondantes.

7. Utilisation des N-oxyles de 4-formylaminopipéridine selon les revendications 1 à 5 pour stabiliser des matériaux organiques vis-à-vis de l'effet de dégradation de radicaux.

8. Utilisation de N-oxyles de 4-formylaminopipéridine selon la revendication 7 pour stabiliser des monomères polymérisables par polymérisation radicalaire.

9. Utilisation de N-oxyles de 4-formylaminopipéridine selon la revendication 8 pour stabiliser le styrène.

10. Procédé de préparation à l'état pur de monomères polymérisables par polymérisation radicalaire par distillation des monomères contaminés, **caractérisé en ce qu'**on procède à la distillation en présence d'un N-oxyle de 4-formylaminopipéridine selon les revendications 1 à 5.

11. Procédé selon la revendication 10, **caractérisé en ce qu'**on l'utilise pour préparer le styrène à l'état pur.

12. Utilisation des N-oxyles de 4-formylaminopipéridine selon les revendications 7 à 10, en même temps que de composés nitrés ou nitrosés aromatiques ou de phénols substitués, ou de mélange de ces composés.

13. Mélanges de N-oxyles de 4-formylaminopipéridine selon les revendications 1 à 5 et de composés nitrés ou nitrosés aromatiques ou de phénols substitués.

14. Matières plastiques, peintures, huiles, graisses et monomères polymérisables par polymérisation radicalaire, contenant un N-oxyle de 4-formylaminopipéridine selon les revendications 1 à 5.
